# EUROPEAN PATENT APPLICATION

(11) **EP 4 667 941 A1**
(43) Date of publication of application: **24.12.2025**
(21) Application number: 24775277.7
(22) Date of filing: 21.03.2024
(51) Int. Cl.: G01N 33/68

(54) **COMPOSITION FOR DIAGNOSING COGNITIVE DYSFUNCTION OF COMPANION ANIMAL BY USING NASAL FLUID**

(30) Priority: 21.03.2023 KR 20230036568
(71) Applicant: Keybasic Co., Ltd, Jeonju-si, Jeonbuk-do 54870 (KR)
(72) Inventor: CHOI, Ju Young, Seongnam-si, Gyeonggi-do 13194 (KR); YOO, Seung-Jun, Seoul 06652 (KR)
(74) Representative: Manitz Finsterwald Patent- und Rechtsanwaltspartnerschaft mbB
(86) International application number: PCT/KR2024/095618
(87) International publication number: WO 2024/196232

(57) **Abstract**

The present disclosure relates to a composition for diagnosing cognitive dysfunction in a companion animal using a nasal fluid sample, and to a kit including the same.

## Description

### TECHNICAL FIELD

The present disclosure relates to a composition for diagnosing cognitive dysfunction in a companion animal using a nasal fluid sample, and to a kit including the same.

### BACKGROUND

Due to demographic changes such as population aging, the rise of nuclear families, and the increase in single-person households, the number of people raising companion animals is growing. According to the 2019 national awareness survey on animal protection conducted by the Ministry of Agriculture, Food and Rural Affairs, it was estimated that in 2019, 5.91 million households in Korea were raising companion animals, accounting for 26.4% of the total households (calculated based on 22.38 million households nationwide). This represented an increase of 0.8 million households from the previous year, reflecting a steady upward trend.

Furthermore, the 2021 Korea companion animal report published by KB Financial Group showed that the total number of companion animal households in 2020 had increased by 120% compared to 2018, and that the number of households raising senior dogs had increased by 179%. Dogs are classified as "senior" at age seven in veterinary medicine, and by the age of eight, the probability of developing canine cognitive dysfunction reaches about one-third of the population. From the age of ten, guardians begin to recognize the aging of their dogs. The research team led by Sarah Yarborough at the Department of Epidemiology, University of Washington (USA), reported in the scientific journal "Scientific Reports" that the risk of dementia in dogs over the age of ten increases by more than 50% with each passing year. When dogs develop cognitive dysfunction, they may show symptoms such as anxiety, disorientation, and inappropriate elimination. This can significantly affect not only the dog's life but also the guardian's. Early diagnosis can help slow the progression of canine cognitive dysfunction. Therefore, there is an urgent need for the development of preventive and early diagnostic methods.

Conventional diagnostic methods for canine cognitive dysfunction include questionnaires such as the CCDR (canine cognitive dysfunction rating scale) and the CADES (canine dementia scale), which are designed to evaluate representative symptoms of canine cognitive dysfunction syndrome. However, as they rely on the guardian's subjective assessment and the veterinarian's limited clinical examination, these methods have limitations in objectivity and reliability. Further, methods such as hematological tests, cytological tests, and imaging examinations require either the collection of brain or spinal tissue for biopsy or the use of anesthesia. These procedures may negatively affect the health of elderly companion animals.

### PRIOR ART DOCUMENT

### Non-patent literature

Hannah E Salvin, Paul D McGreevy, Perminder S Sachdev, Michael J Valenzuela, "The canine cognitive dysfunction rating scale (CCDR): a data-driven and ecologically relevant assessment tool", Vet J. 2011 Jun;188(3):331-6.

### DISCLOSURE OF THE INVENTION

### PROBLEMS TO BE SOLVED BY THE INVENTION

The present disclosure provides a composition for diagnosing cognitive dysfunction in a companion animal using a nasal fluid sample, and a kit containing the same.

However, problems to be solved by the present disclosure are not limited to the above-described problems, and although not described herein, other problems to be solved by the present disclosure can be clearly understood by those skilled in the art from the following descriptions.

### MEANS FOR SOLVING THE PROBLEMS

A first aspect of the present disclosure provides a composition for diagnosing cognitive dysfunction in a companion animal, including: an agent for measuring an expression level of at least one protein selected from UGT8 and BUD31, a fragment thereof, or a gene encoding the protein.

A second aspect of the present disclosure provides a kit for diagnosing cognitive dysfunction in a companion animal, including: the composition according to Claim 1.

A third aspect of the present disclosure provides a method for diagnosing cognitive dysfunction in a companion animal, including:
(a) a process of measuring an expression level of at least one protein selected from UGT8 and BUD31, a fragment thereof, or a gene encoding the protein, in a sample obtained from a subject; and
(b) a process of comparing the measured expression level to an expression level in a control group.

### EFFECTS OF THE INVENTION

According to embodiments of the present disclosure, a composition for diagnosing cognitive dysfunction in a companion animal and a kit containing the same include an agent for detecting a protein capable of diagnosing cognitive dysfunction in a companion animal, a fragment thereof, or a gene encoding the protein. This kit allows a guardian to conveniently diagnose cognitive dysfunction in a companion animal using a nasal fluid sample. Therefore, diagnosis can be performed with reduced time and expense compared to a visit to a veterinary hospital.

According to embodiments of the present disclosure, the composition for diagnosing cognitive dysfunction in a companion animal and the kit containing the same enable non-invasive diagnosis of cognitive dysfunction in a companion animal.

According to embodiments of the present disclosure, the composition for diagnosing cognitive dysfunction in a companion animal and the kit containing the same can be conveniently used without temporal or spatial restrictions and thus enable early diagnosis of cognitive dysfunction in a companion animal.

### BRIEF DESCRIPTION OF THE DRAWINGS

**FIG. 1A** and **FIG. 1B** illustrate Western blot bands for UGT8 and BUD31, respectively, according to an example of the present disclosure.
**FIG. 2A** and **FIG. 2B** are graphs quantitatively representing the Western blot bands for UGT8 and BUD31, respectively, according to an example of the present disclosure.

### BEST MODE FOR CARRYING OUT THE INVENTION

Hereinafter, embodiments and examples of the present disclosure will be described in detail with reference to the accompanying drawings so that the present disclosure may be readily implemented by those skilled in the art. However, it is to be noted that the present disclosure is not limited to the embodiments and examples but can be embodied in various other ways. In drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element.

Through the whole document, the term "on" that is used to designate a position of one element with respect to another element includes both a case that the one element is adjacent to the other element and a case that any other element exists between these two elements.

Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Through the whole document, the term "about or approximately" or "substantially" is intended to have meanings close to numerical values or ranges specified with an allowable error and intended to prevent accurate or absolute numerical values disclosed for understanding of the present disclosure from being illegally or unfairly used by any unconscionable third party.

Through the whole document, the term "step of" does not mean "step for".

Through the whole document, the term "combination(s) of" included in Markush type description means mixture or combination of one or more components, steps, operations and/or elements selected from a group consisting of components, steps, operation and/or elements described in Markush type and thereby means that the disclosure includes one or more components, steps, operations and/or elements selected from the Markush group.

Through the whole document, a phrase in the form "A and/or B" means "A or B, or A and B".

Throughout the whole document, the term "nasal fluid sample" may include, for example, a sample obtained by spraying saline into the nasal cavity and then collecting it with a nasal aspirator, or by flushing saline into the nose and collecting the flowing nasal discharge.

Throughout the whole document, the term "companion animal" refers to animals raised for companionship without limitation, and non-limiting examples include dogs, cats, hamsters, rabbits, and guinea pigs.

Throughout the whole document, the term "biomarker" refers to an indicator, such as a gene or protein, that can be used to predict the onset, likelihood of onset, or progression of a particular disease in a biological sample obtained from a target subject.

Throughout the whole document, the term "cognitive dysfunction" may refer to "cognitive impairment" or "cognitive dysfunction syndrome."

Hereinafter, embodiments of the present disclosure are described in detail. However, the present disclosure is not limited thereto.

A first aspect of the present disclosure provides a composition for diagnosing cognitive dysfunction in a companion animal, including: an agent for measuring an expression level of at least one protein selected from UGT8 and BUD31, a fragment thereof, or a gene encoding the protein.

UGT8 (UDP glycosyltransferase 8) is 2-hydroxyacylsphingosine 1-beta-galactosyltransferase, and the nucleotide sequence of the gene is shown in SEQ ID No: 1. Since the nucleotide sequence of the gene is registered in a gene bank, a person with ordinary skill in the art can readily obtain it (NCBI Gene ID: 7368).

The UGT8 protein encoded by the UGT8 gene includes isoforms or precursors thereof. However, a representative amino acid sequence of the protein is shown in SEQ ID No: 2 in the present disclosure.

BUD31 (BUD31 Homolog) is part of the U2-type catalytic step 2 spliceosome, and the nucleotide sequence of the gene is shown in SEQ ID No: 3. Since the nucleotide sequence of the gene is registered in a gene bank, a person with ordinary skill in the art can readily obtain it (NCBI Gene ID: 8896).

The BUD31 protein encoded by the BUD31 gene includes isoforms or precursors thereof. However, a representative amino acid sequence of the protein is shown in SEQ ID No: 4 in the present disclosure.

### [UGT8 Sequence listing]

<SEQ ID No: 1>
   (https://www.ncbi.nlm.nih.gov/nuccore/NC_000004.12?from=114598402&to= 114678225&report=genbank)
<SEQ ID No: 2>

mksytpyfml lwsavgiaka akiiivppim feshmyifkt lasalhergh rtvfllsegr diapsnhysl
qrypgifnst tsdaflqskm rnifsgrlta ielfdildhy tkncdmmvgn raliqglkke kfdlllvdpn dmcgfviahl lgvkyavfst
glwypaevga paplayvpef nslltdhmnl lqrmkntgvy lisrlgvsfl vlpkyerimq kynllpeksm ydlvhgsslw mlctdvalef
prptlpnvvy vggiltkpag plpedlqrwv nganehgfvl vsfgagvkyl sedianklag algrlpqkvi wrfsgtkpkn lgnntkliew
lpqndllghs nikaflshgg lnsifetmyh gvpvvgiplf gdhydtmtrv qakgmgille wktvtegely ealvkvinnp syrqraqkls
eihkdqpghp vnrtvywidy ilrhngahhl raavhqisfc qyflldiafv lllgaalfyf flswvtkfiy rkikslwsrn khstvnghyh
ngilngkykr nghikhekkv k

### [BUD31 Sequence listing]

<SEQ ID No: 3>
   (https://www.ncbi.nlm.nih.gov/nuccore/NC_000007.14?from=99408969&to=9 9419616&report=genbank)
<SEQ ID No: 4>

mpkvkrsrka ppdgweliep tldeldqkmr eaetephegk rkveslwpif rihhqktryi fdlfykrkai

srelyeycik egyadknlia kwkkqgyenl cclrciqtrd tnfgtncicr vpksklevgr iiecthcgcr gcsg

In an embodiment of the present disclosure, the companion animal may include a dog, cat, hamster, rabbit, or guinea pig, but is not limited thereto.

In an embodiment of the present disclosure, the expression level of the protein, the fragment thereof, or the gene may be measured from at least one selected from nasal fluid, saliva, blood, plasma, serum, urine, mucus, tears, sputum, cerebrospinal fluid, pleural effusion, nipple aspirate fluid, lymph, airway fluid, serous fluid, urogenital tract fluid, breast milk, lymphatic fluid, semen, cerebrospinal fluid, organ system fluid, ascites, cystic tumor fluid, and amniotic fluid of a companion animal.

In an embodiment of the present disclosure, the expression level of the protein, the fragment thereof, or the gene may be measured from nasal fluid and/or saliva of a companion animal, but is not limited thereto.

In an embodiment of the present disclosure, the protein, the fragment thereof, or the gene is a protein or gene specifically expressed upon the onset of cognitive dysfunction in a companion animal and may be contained in nasal fluid or saliva of the companion animal.

Throughout the whole document, the term "measurement of protein expression level" refers to a process of confirming the presence and expression level of a biomarker protein in a subject sample, and may include measuring the amount of the biomarker protein. Alternatively, it may include measuring the expression level of a polynucleotide, such as mRNA, that encodes the biomarker protein.

In an embodiment of the present disclosure, the agent may detect the presence of the protein and may also measure the expression level of the protein.

In an embodiment of the present disclosure, the expression level of the protein, the fragment thereof, or the gene may be increased compared to the expression level in a normal control group.

In an embodiment of the present disclosure, in a companion animal suffering from cognitive dysfunction, the expression level of the UGT8 gene or the protein expressed from the UGT8 gene may be increased about 500-fold to about 4000-fold compared to a normal control group.

In an embodiment of the present disclosure, in a companion animal suffering from cognitive dysfunction, the expression level of the BUD31 gene or the protein expressed from the BUD31 gene may be increased about 100-fold to about 4000-fold compared to a normal control group.

In an embodiment of the present disclosure, the agent for measuring the expression level of the gene may include at least one selected from primers, probes, and antisense nucleotides specifically binding to a polynucleotide encoding the protein, but is not limited thereto.

In an embodiment of the present disclosure, the agent for measuring the expression level of the protein or the fragment thereof may include at least one selected from antibodies, antigen-binding fragments thereof, oligopeptides, ligands, peptide nucleic acids (PNAs), and aptamers specifically binding to the protein or the fragment thereof, but is not limited thereto.

Typically, the term "antibody" may refer to a specific protein molecule directed against an antigenic site.

In an embodiment of the present disclosure, the antibody means an antibody specifically binding to a protein capable of diagnosing cognitive dysfunction in the companion animal, and may include polyclonal antibodies, monoclonal antibodies, and recombinant antibodies. The antibody can be easily produced using a technique widely known in the art. Polyclonal antibodies can be produced by injecting a target protein antigen into an animal and collecting serum containing an antibody from the animal according to a widely known method. Such polyclonal antibodies can be prepared from a host derived from any animal species such as goat, rabbit, sheep, monkey, horse, pig, cow, or dog. Monoclonal antibodies can be produced using a method well known in the art such as a hybridoma method (see Kohler and Milstein (1976) European Journal of Immunology 6:511-519), or phage antibody libraries (Clackson et al, Nature, 352:624-628, 1991; Marks et al, J. Mol. Biol., 222:58, 1-597, 1991). Antibodies produced by these methods can be separated and purified using methods such as gel electrophoresis, dialysis, salt precipitation, ion-exchange chromatography, and affinity chromatography. The antibody prepared by the above-described method can be isolated and purified using a method such as gel electrophoresis, dialysis, salt precipitation, ion exchange chromatography, affinity chromatography, etc.

In an embodiment of the present disclosure, the antibody may include a functional fragment of an antibody molecule, as well as a complete form having two full-length light chains and two full-length heavy chains. The functional fragment of the antibody molecule refers to a fragment having at least antigen-binding function, and may include Fab, F(ab'), F(ab')₂, and Fv.

In an embodiment of the present disclosure, analytical methods for confirming the amount of target protein bound by the antibody may include Western blotting, enzyme-linked immunosorbent assay (ELISA), radioimmunoassay (RIA), radioimmunodiffusion, Ouchterlony immunodiffusion, rocket immunoelectrophoresis, immunohistochemistry, immunoprecipitation assay, complement fixation assay, fluorescence-activated cell sorting (FACS), and protein chip, but are not limited thereto.

Typically, the term "aptamer" may refer to a single-stranded DNA (ssDNA) or RNA having high specificity and affinity to a specific substance. Aptamers have very high affinity and stability, and can be synthesized by relatively simple methods. Also, they can be modified in various ways to increase binding capacity, and can target cells, proteins, and small organic substances. Accordingly, their specificity and stability are much higher than those of conventional antibodies.

In an embodiment of the present disclosure, the aptamer is not limited as long as it can specifically bind to the β-amyloid oligomer and/or the second protein. Unless otherwise indicated, the bases used in the aptamers may be selected from the group consisting of A, G, C, U, and their deoxy forms.

In an embodiment of the present disclosure, the process of measuring the expression level of the protein or fragment thereof may be performed by Western blotting, electrophoresis, enzyme-linked immunosorbent assay (ELISA), immunohistochemistry, protein chip, immunoprecipitation, mass spectrometry, or combinations thereof; and/or the process of measuring the expression level of the gene may be performed by RT-PCR, competitive RT-PCR, real-time RT-PCR, RNase protection assay (RPA), Northern blotting, DNA chip, or combinations thereof.

In an embodiment of the present disclosure, the cognitive dysfunction in the companion animal may include at least one selected from mild cognitive dysfunction, moderate cognitive dysfunction, mild Alzheimer's disease, and moderate Alzheimer's disease, but is not limited thereto.

A second aspect of the present disclosure provides a kit for diagnosing cognitive dysfunction in a companion animal, including: the composition according to Claim 1.

Detailed descriptions of the second aspect of the present disclosure, which overlap with those of the first aspect of the present disclosure, are omitted hereinafter, but the descriptions of the first aspect of the present disclosure may be identically applied to the second aspect of the present disclosure, even though they are omitted hereinafter.

In an embodiment of the present disclosure, the kit may include, in addition to the agent for detecting a protein capable of diagnosing cognitive dysfunction in a companion animal or measuring the expression level thereof in a nasal fluid sample of the companion animal, one or more compositions, solutions, or devices suitable for expression level analysis. For example, the kit may include substrates, appropriate buffer solutions, secondary antibodies labeled with a detectable marker, and chromogenic substrates for immunological detection of antibodies.

In an embodiment of the present disclosure, the agent may be selected from antibodies, peptides, aptamers, and compounds that specifically bind to a protein capable of diagnosing cognitive dysfunction in a companion animal, and may be included in the kit in the form of a protein chip containing the agent. The kit may further include a secondary antibody capable of detecting the antibody or aptamer.

In an embodiment of the present disclosure, measuring the expression level of the protein or fragment thereof in the kit may be implemented by Western blotting, electrophoresis, enzyme-linked immunosorbent assay (ELISA), immunohistochemistry, protein chip, immunoprecipitation, mass spectrometry, or combinations thereof; and/or measuring the expression level of the gene may be implemented by RT-PCR, competitive RT-PCR, real-time RT-PCR, RNase protection assay (RPA), Northern blotting, DNA chip, or combinations thereof.

In an embodiment of the present disclosure, the kit may include an ELISA kit and essential components for performing various ELISA methods such as sandwich ELISA. In an embodiment of the present disclosure, the ELISA kit may include an agent that specifically binds to the protein and a reagent that detects the bound agent. For example, the reagent may include labeled secondary antibodies, chromophores, enzymes and their substrates, or other substances that bind to antibodies.

In an embodiment of the present disclosure, the kit may be a kit for implementing Western blotting, ELISA, immunoprecipitation assay, complement fixation assay, fluorescence-activated cell sorting (FACS), or protein chip, and may further include additional components suitable for each analytical method. Through these analytical methods, the diagnosis of cognitive dysfunction and screening of disease progression in a companion animal can be achieved by comparing the amount of antigen-antibody complex, and customized treatment methods may be provided according to the stage of progression.

In an embodiment of the present disclosure, the composition for diagnosing cognitive dysfunction in a companion animal and the kit containing the same use a nasal fluid sample of the companion animal. The composition has a higher protein expression level than other samples, such as blood and cerebrospinal fluid, thereby facilitating diagnosis. More specifically, among companion animals, dogs have more than 15 times as many olfactory epithelial cells as humans. Therefore, it may be easier to measure the protein expression level using a nasal fluid sample.

A third aspect of the present disclosure provides a method for diagnosing cognitive dysfunction in a companion animal, including:
(a) a process of measuring an expression level of at least one protein selected from UGT8 and BUD31, a fragment thereof, or a gene encoding the protein, in a sample obtained from a subject; and
(b) a process of comparing the measured expression level to an expression level in a control group.

Detailed descriptions of the third aspect of the present disclosure, which overlap with those of the first and second aspects of the present disclosure, are omitted hereinafter, but the descriptions of the first and second aspects of the present disclosure may be identically applied to the third aspect of the present disclosure, even though they are omitted hereinafter.

In an embodiment of the present disclosure, the sample may include at least one selected from nasal fluid, saliva, blood, plasma, serum, urine, mucus, tears, sputum, cerebrospinal fluid, pleural effusion, nipple aspirate fluid, lymph, airway fluid, serous fluid, urogenital tract fluid, breast milk, lymphatic fluid, semen, cerebrospinal fluid, organ system fluid, ascites, cystic tumor fluid, and amniotic fluid of a companion animal.

In an embodiment of the present disclosure, the sample may include nasal fluid and/or saliva of a companion animal.

In an embodiment of the present disclosure, the process of measuring the expression level of the protein or the fragment thereof may be performed by Western blotting, electrophoresis, enzyme-linked immunosorbent assay (ELISA), immunohistochemistry, protein chip, immunoprecipitation, mass spectrometry, or combinations thereof; and/or the process of measuring the expression level of the gene may be performed by RT-PCR, competitive RT-PCR, real-time RT-PCR, RNase protection assay (RPA), Northern blotting, DNA chip, or combinations thereof.

Hereinafter, the present disclosure will be explained in more detail with reference to Examples. However, the following Examples are illustrative only for better understanding of the present disclosure but do not limit the present disclosure.

### MODE FOR CARRYING OUT THE INVENTION

### 1. Sampling for Biomarker Validation and Discovery

To validate and discover biomarkers, samples were designed and collected. The definition of a dog with cognitive dysfunction was referenced from "The canine cognitive dysfunction rating scale (CCDR): a data-driven and ecologically relevant assessment tool" (Vet. J. 2011 Jun;188(3):331-336). Considering the tendency for prevalence in dogs to increase with age, a suitable reference age was determined. A test subject was limited to Beagle, Poodle, Maltese, Chihuahua, Yorkshire Terrier, Dachshund, Spaniel, and Schnauzer with reference to the paper "Biomarkers for Early Diagnosis of Degenerative Genetic Diseases in Companion Dogs." Information on breed, body weight, age, and diseases of the test subject was collected to prepare a diagnostic chart based on CCDR items. Thereafter, the subject population was grouped. Group 1 was composed of dogs aged 10 years or older with cognitive dysfunction. In this group, nasal fluid and blood were collected from 10 dogs, and nasal fluid, blood, and cerebrospinal fluid (CSF) were collected from 3 dogs. Group 2 was composed of dogs aged 10 years or older with cranial neurological disease. In this group, nasal fluid and blood were collected from 10 dogs, and nasal fluid, blood, and CSF were collected from 3 dogs. Group 3 was composed of normal dogs aged 10 years or older. In this group, nasal fluid and blood were collected from 5 dogs, and nasal fluid, blood, and CSF were collected from 5 dogs. Group 4 was composed of normal dogs aged 10 years or older. In this group, nasal fluid and blood were collected from 5 dogs, and nasal fluid, blood, and CSF were collected from 3 dogs. The collection of each sample was conducted in accordance with the Institutional Animal Care and Use Committee (IACUC) guidelines. Nasal fluid was collected by scraping the nasal epithelium (a small admixture of blood may occur), blood was processed to separate serum/plasma, and CSF was collected at about 1 mL per animal, with a minimum of 0.3 mL. The samples were immediately frozen at -20°C to -80°C after collection and were storable for up to about 3 months after freezing.

### 2. Identification of Candidate Biomarker

Proteomics analysis of samples collected from the cognitive dysfunction groups (Groups 1 and 2) and the normal groups (Groups 3 and 4) was performed to identify significant proteins (candidate biomarkers) by category (Table 1).

**[Table 1]**

| | Category | Protein |
|---|---|---|
| 1 | metabolic process | UGT8 |
| 2 | gene expression process | BUD31 |
| 3 | cellular/extracellular component | TOMM34 |
| 4 | metabolic process and cellular/extracellular component | SRP14 |

### 3. Selection of Biomarker

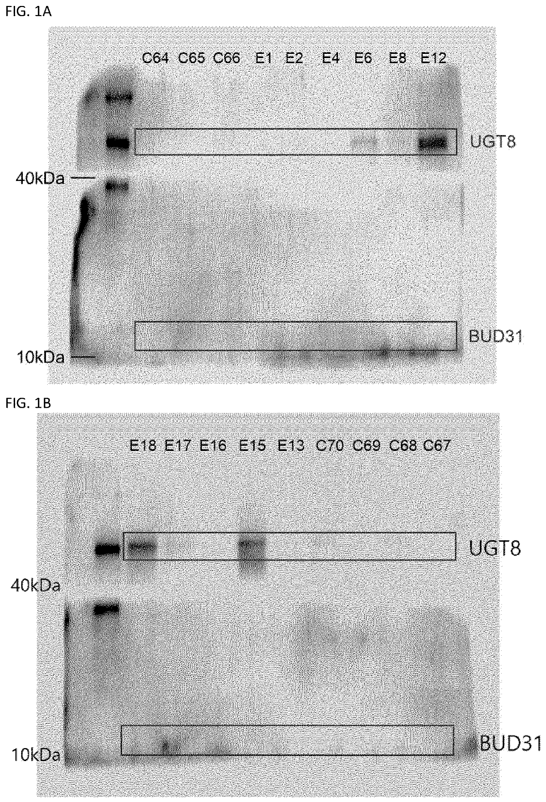

Western blotting was employed to select a biomarker from the candidate biomarkers UGT8, BUD31, TOMM34, and SRP14. The test samples were nasal cavity samples obtained from 11 dogs with cognitive dysfunction syndrome (CDS) and 7 dogs in a normal control group. As a result, it was visually confirmed that proteins UGT8 and BUD31 were significantly detected in the CDS group (Sample Nos.: E1, E2, E4, E6, E8, E12, E13, E15, E16, E17, and E18) compared to the normal control group (Sample Nos.: C64, C65, C66, C67, C68, C69, and C70) (**FIG. 1A** and **FIG. 1B**). Accordingly, UGT8 and BUD31 can be selected as positive biomarkers.

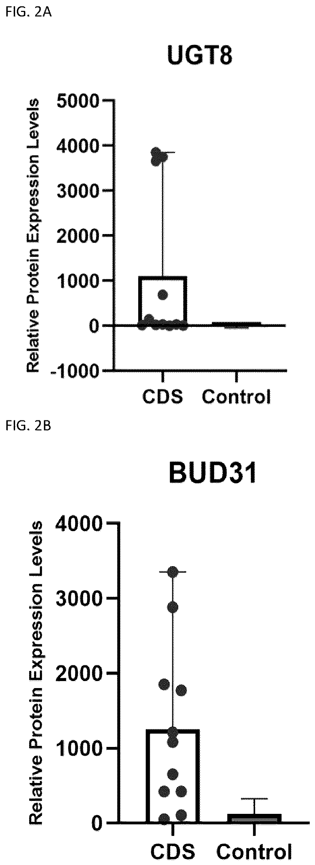

The relative intensities of the Western blot bands for UGT8 and BUD31 were quantified and graphed (**FIG. 2A** and **FIG. 2B**)**.** For both UGT8 and BUD31, it was confirmed that the protein expression levels of the positive biomarkers were significantly higher in the CDS group than in the normal control group. In particular, referring to **FIG. 2B****,** for the BUD31 protein, the inter-sample variance was smaller and the expression level was higher in the CDS group than in the normal control group.

### 4. Performance Indicator

First, as shown in Table 2 below, the numbers of true positives (TP), false negatives (FN), false positives (FP), and true negatives (TN) were obtained for UGT8 and BUD31.

**[Table 2]**

| Category | | Predicted values | |
|---|---|---|---|
| | | Ture | False |
| Actual values | Positive | TP | FN |
| | Negative | FP | TN |

That is, in Table 2, the actual positives are the numbers of dogs diagnosed as positive by the canine cognitive dysfunction assessment (DISHAA assessment), and TP and FN denote the numbers of dogs diagnosed as positive and negative, respectively, by the biomarkers in the examples of the present disclosure. Further, the actual negatives are the numbers of dogs diagnosed as negative by the DISHAA assessment, and FP and TN denote the numbers of dogs diagnosed as positive and negative, respectively, by the biomarkers in the examples of the present disclosure.

Tables 3 and 4 summarize the numbers of TP, FN, FP, and TN for UGT8 and BUD31. For UGT8 and BUD31, a band intensity of 100 or more was considered positive, and less than 100 was considered negative.

**[Table 3]**

| UGT8 | | Predicted values | |
|---|---|---|---|
| | | Ture | False |
| Actual values | Positive | TP : 6 | FN : 5 |
| | Negative | FP : 0 | TN : 7 |

**[Table 4]**

| BUD31 | | Predicted values | |
|---|---|---|---|
| | | Ture | False |
| Actual values | Positive | TP:8 | FN : 3 |
| | Negative | FP : 0 | TN : 7 |

Further, according to the formulas in Table 5, performance indicators (sensitivity, specificity, precision, recall, and accuracy) were calculated to evaluate whether the selected biomarkers are applicable to practical diagnosis.

**[Table 5]**

| | |
|---|---|
| Sensitivity | (TP / TP+FN) |
| | Number of positives correctly predicted as positive |
| Specificity | (TN / FP+TN) |
| | Number of negatives correctly predicted as negative |
| Precision | (TP / TP+FP) |
| | Number of actual positives among those predicted positive |
| Recall | (TP / TP+FN) |
| | Number of detected positives among all positives |
| Accuracy | (TP+TN / TP+FN+FP+TN) |
| | Number of correctly predicted positives and negatives out of the total |

**[Table 6]**

| Performance indicators | Biomarker | |
|---|---|---|
| | UGT8 | BUD31 |
| Sensitivity | 54.5% | 72.7% |
| Specificity | 100% | 100% |
| Precision | 100% | 100% |
| Recall | 54.5% | 72.7% |
| Accuracy | 72.2% | 83.3% |

Since the performance indicators for each biomarker exceeded 70%, it was confirmed that the biomarkers of the present disclosure are applicable to practical diagnosis.

The above description of the present disclosure is provided for the purpose of illustration, and it would be understood by those skilled in the art that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A composition for diagnosing cognitive dysfunction in a companion animal, comprising:
an agent for measuring an expression level of at least one protein selected from UGT8 and BUD31, a fragment thereof, or a gene encoding the protein.

2. The composition of Claim 1,
wherein the expression level of the protein, the fragment thereof, or the gene is measured from at least one selected from nasal fluid, saliva, blood, plasma, serum, urine, mucus, tears, sputum, cerebrospinal fluid, pleural effusion, nipple aspirate fluid, lymph, airway fluid, serous fluid, urogenital tract fluid, breast milk, lymphatic fluid, semen, cerebrospinal fluid, organ system fluid, ascites, cystic tumor fluid, and amniotic fluid of a companion animal.

3. The composition of Claim 1,
wherein the expression level of the protein, the fragment thereof, or the gene is measured from nasal fluid and/or saliva of a companion animal.

4. The composition of Claim 1,
wherein the expression level of the protein, the fragment thereof, or the gene can be increased compared to the expression level in a normal control group.

5. The composition of Claim 1,
wherein the agent for measuring the expression level of the gene includes at least one selected from primers, probes, and antisense nucleotides specifically binding to a polynucleotide encoding the protein, but is not limited thereto.

6. The composition of Claim 1,
wherein the agent for measuring the expression level of the protein or the fragment thereof includes at least one selected from antibodies, antigen-binding fragments thereof, oligopeptides, ligands, peptide nucleic acids (PNAs), and aptamers specifically binding to the protein or the fragment thereof.

7. The composition of Claim 1,
wherein the cognitive dysfunction in the companion animal includes at least one selected from mild cognitive dysfunction, moderate cognitive dysfunction, mild Alzheimer's disease, and moderate Alzheimer's disease.

8. A kit for diagnosing cognitive dysfunction in a companion animal, comprising the composition according to Claim 1.

9. A method for diagnosing cognitive dysfunction in a companion animal, comprising:
(a) a process of measuring the expression level of at least one protein selected from UGT8 and BUD31, a fragment thereof, or a gene encoding the protein, in a sample obtained from a subject; and
(b) a process of comparing the measured expression level to an expression level in a control group.

10. The method of Claim 9,
wherein the sample includes at least one selected from nasal fluid, saliva, blood, plasma, serum, urine, mucus, tears, sputum, cerebrospinal fluid, pleural effusion, nipple aspirate fluid, lymph, airway fluid, serous fluid, urogenital tract fluid, breast milk, lymphatic fluid, semen, cerebrospinal fluid, organ system fluid, ascites, cystic tumor fluid, and amniotic fluid of a companion animal.

11. The method of Claim 9,
wherein the process of measuring the expression level of the protein or the fragment thereof is performed by Western blotting, electrophoresis, enzyme-linked immunosorbent assay (ELISA), immunohistochemistry, protein chip, immunoprecipitation, mass spectrometry, or combinations thereof; and/or
the process of measuring the expression level of the gene is performed by RT-PCR, competitive RT-PCR, real-time RT-PCR, RNase protection assay (RPA), Northern blotting, DNA chip, or combinations thereof.
